(19) Europäisches Patentamt
European Patent Office
Office européen des brevets

(11) Veröffentlichungsnummer: **0 050 871**
**B1**

# (12) EUROPÄISCHE PATENTSCHRIFT

(45) Veröffentlichungstag der Patentschrift: **09.05.84**

(51) Int. Cl.³: **C 07 C 87/02,** C 07 C 85/20, C 07 C 69/06, C 07 C 67/22

(21) Anmeldenummer: **81108900.2**

(22) Anmeldetag: **25.10.81**

**(54) Verfahren zur Herstellung von N-tert.-Alkylaminen oder Cycloalkylaminen und Estern der Ameisensäure.**

(30) Priorität: **27.10.80 DE 3040404**

(43) Veröffentlichungstag der Anmeldung: **05.05.82 Patentblatt 82/18**

(45) Bekanntmachung des Hinweises auf die Patenterteilung: **09.05.84 Patentblatt 84/19**

(84) Benannte Vertragsstaaten: **AT BE CH DE FR GB LI NL**

(56) Entgegenhaltungen:
**DE - B - 1 197 091**
**DE - B - 1 493 433**

(73) Patentinhaber: **Degussa Aktiengesellschaft, Weissfrauenstrasse 9, D-6000 Frankfurt am Main 1 (DE)**

(72) Erfinder: **Kleemann, Axel, Dr. Dipl.-Chem., Greifenhagenstrasse 9, D-6450 Hanau 9 (DE)**
Erfinder: **Schalke, Peter, Dr. Dipl.-Chem., Südring 74, D-6458 Rodenbach (DE)**
Erfinder: **Lehmann, Bernd, Dr. Dipl.-Chem., Flurstrasse 5, D-6463 Freigericht 1 (DE)**

**EP 0 050 871 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Aminen und Estern unter Umsetzung von Alkenen mit Cyanwasserstoff in Gegenwart starker Säuren.

Es sind mehrere Verfahren zur Herstellung von N-tert.-Alkylaminen bekannt. In allen Fällen werden zunächst in einer ersten Verfahrensstufe N-tert.-Alkylformamide gewonnen. Aus diesen werden dann in einer zweiten Verfahrensstufe durch Hydrolyse die N-tert.-Alkylamine erzeugt, wobei gleichzeitig Ameisensäure entsteht.

Bekannt ist, dass bei der Umsetzung von Alkenen mit Cyanwasserstoff in Gegenwart von stark sauren Kondensationsmitteln, wie Schwefelsäure, Formamide entstehen und diese durch Hydrolyse mittels starker Säuren oder Basen in die entsprechenden Amine und Ameisensäure übergeführt werden. Beispielsweise wird aus Isobuten das N-tert.-Butylformamid und aus diesem neben Ameisensäure das N-tert.-Butylamin gebildet. Im Falle der Hydrolyse des Formamids mittels Chlorwasserstoffsäure wird aus dem Umsetzungsgemisch zunächst die Ameisensäure und dann nach Zugabe von Basen das Amin abdestilliert (DE-PS 870 856). Nachteilig ist bei diesem Verfahren, dass die Ausbeuten mässig sind und überdies die Ameisensäure in Form unreiner verdünnter wässriger Lösungen anfällt.

Es ist auch bekannt, N-tert.-Alkylformamide durch Umsetzung tertiärer Alkohole oder verzweigter Alkene mit Cyanwasserstoff unter Verwendung von Ameisensäure als Kondensationsmittel zu erzeugen und die Formamide durch Hydrolyse mittels Alkali in die N-tert.-Alkylamine zu überführen (DE-AS 22 36 040). Bei diesem Verfahren sind die Umsetzungszeiten für die Bildung der Formamide sehr lang und die erzielten Raum-Zeit-Ausbeuten gering. Brauchbare Ausbeuten werden nur erreicht, wenn der Cyanwasserstoff und insbesondere die Ameisensäure in mehrfach molarem Überschuss eingesetzt werden. Diese im Überschuss angewendeten Substanzen können nur unter erheblichem Aufwand und überdies nur unvollständig zurückgewonnen werden.

Bekannt ist ausserdem, dass aus tert.-Alkyläthern die entsprechenden N-tert.-Alkylformamide entstehen, wenn die Äther mit Cyanwasserstoff in der 4fachen bis 50fachen Menge konzentrierter Säure umgesetzt werden und das Umsetzungsgemisch mit Wasser verdünnt wird (US-PS 2 518 156). Dieses Verfahren ist wegen der erforderlichen grossen Menge konzentrierter Säure sehr aufwendig.

Schliesslich ist auch bekannt, das durch Umsetzung von Alkenen mit Cyanwasserstoff in Gegenwart von Schwefelsäure erzeugte tert.-Butylformamid mittels Natriumhydroxid zu hydrolysieren, zunächst aus dem Umsetzungsgemisch das tert.-Butylamin abzudestillieren, dann in dem restlichen Umsetzungsgemisch die Ameisensäure mittels Schwefelsäure freizusetzen und mit Methanol zu verestern und schliesslich den Ameisensäuremethylester abzudestillieren (US-PS 4 131 642). Bei diesem Verfahren ist die Ausbeute sowohl an dem tert.-Butylamin als auch an dem Ameisensäureester unbefriedigend.

Es ist nun ein Verfahren zur Herstellung von Aminen und Estern unter Umsetzung von Alkenen mit Cyanwasserstoff in Gegenwart starker Säuren gefunden worden, das dadurch gekennzeichnet ist, dass Alkene, die an mindestens einer Seite der Doppelbindung verzweigt sind, oder Cycloalkene in Gegenwart der bis zu etwa zweifachen molaren Menge der starken Säuren mittels primärer oder sekundärer Alkanole zu N-tert.-Alkylaminen oder Cycloalkylaminen und Estern der Ameisensäure mit primären oder sekundären Alkanolen umgesetzt werden. Bei diesem Verfahren werden unmittelbar aus den Alkenen die Amine und gleichzeitig die Ester der Ameisensäure gebildet. Sowohl die Amine als auch die Ester fallen mit ausgezeichneter Ausbeute an.

Das erfindungsgemässe Verfahren ist besonders geeignet für die Umsetzung von Alkenen der allgemeinen Formel

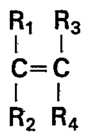

I

in der $R_1$, $R_2$, $R_3$ und $R_4$ gleich oder verschieden sind und

a) $R_1$ und $R_2$ unverzweigte oder verzweigte, gegebenenfalls substituierte, Alkylgruppen mit 1 bis 10, vorzugsweise 1 bis 4, Kohlenstoffatomen, insbesondere Methylgruppen, bedeuten oder gemeinsam zu einem, gegebenenfalls substituierten, Cycloalkylring verbunden sind, und $R_3$ und $R_4$ Wasserstoff oder, gegebenenfalls substituierte, Alkylgruppen mit 1 bis 10, vorzugsweise 1 bis 4, Kohlenstoffatomen bedeuten, und insbesondere $R_3$ Wasserstoff und $R_4$ Wasserstoff oder eine Methylgruppe ist, und

b) $R_1$ und $R_3$ gemeinsam mit der $>C=C<$-Gruppe zu einem, gegebenenfalls substituierten, Cycloalkenylring mit insgesamt 15 bis 12, vorzugsweise 5 bis 8, Kohlenstoffatomen im Ring, insbesondere zu einem Cyclohexenring, verbunden sind und $R_2$ und $R_4$ Wasserstoff oder gegebenenfalls substituierte, Alkylgruppen mit 1 bis 10, vorzugsweise 1 bis 4, Kohlenstoffatomen, insbesondere Wasserstoff oder Methylgruppen, bedeuten.

mittels Alkanolen der allgemeinen Formel

$$R_5\text{–}OH \qquad \text{II}$$

in der $R_5$ eine unverzweigte oder verzweigte Alkylgruppe mit 1 bis 10 Kohlenstoffatomen, vorzugsweise eine Propylgruppe, Isopropylgruppe oder Äthylgruppe, insbesondere eine Methylgruppe, ist, mit Cyanwasserstoff zu Aminen der allgemeinen Formel

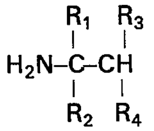

III

in der $R_1$, $R_2$, $R_3$ und $R_4$ die zuvor angegebenen Bedeutungen haben, und Ameisensäureestern der allgemeinen Formel

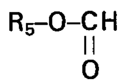

IV

in der $R_5$ die zuvor angegebenen Bedeutungen hat.

Als Alkene kommen beispielsweise 2-Methylpenten-(1), 2-Methylhexen-(1), 3-Methylhexen-(3), 2,3-Dimethylbuten-(2), 2,3-Dimethylpenten-(1), 2,3-Dimethylhexen-(1), 2,4-Dimethylhexen-(1), 2,5-Dimethylhexen-(1), 2-Methylhepten-(1), 2-Methylhepten-(2), 2,5-Dimethylhepten-(1), 2,5-Dimethylhepten-(2), 2,3-Diäthylhexen-(1), 2,3-Diäthylhexen-(2), 2-Methyl-3-äthylpenten-(1), 2,4,4-Trimethylpenten-(2), 2, 3, 3-Trimethylhepten-(1), 2, 3, 3, 4-Tetramethylhexen-(1), 2,5-Dimethylhexadien-(2,4), Camphen, Pinen, Cyclopenten, Cyclohexen, Cyclohepten, Cyclooсten, 1-Methylcyclohexen, 4-Methylcyclohexen, 1-Methylcyclopenten, Limonen und Norbornen in Frage. Mit besonderem Vorteil ist das erfindungsgemässe Verfahren für die Umsetzung von 2-Methylbuten-(1), 2-Methylpenten-(2), insbesondere von 2-Methylbuten-(2) und Isobuten, geeignet.

Statt Cyanwasserstoff können Substanzen verwendet werden, die unter den Umsetzungsbedingungen Cyanwasserstoff liefern, beispielsweise Salze der Cyanwasserstoffsäure, wie Natriumcyanid.

Als starke Säuren werden beispielsweise Monoester der Schwefelsäure, zweckmässigerweise mit einem Alkanol, das der allgemeinen Formel

$R_5$–OH II

entspricht, in der $R_5$ die zuvor genannten Bedeutungen hat, oder Sulfonsäuren, wie Methansulfonsäure oder Benzolsulfonsäure, eingesetzt. Besonders geeignet ist Schwefelsäure.

In welchen Mengenverhältnissen die Substanzen angewendet werden und welche Umsetzungsbedingungen, wie Temperatur und Druck, gewählt werden, richtet sich gegebenenfalls nach der Art der Substanzen.

Je Mol des Alkens werden bis zu etwa 2 Mol der starken Säuren eingesetzt. Im allgemeinen ist es zweckmässig, etwa 0,8 bis 1,8 Mol der Säuren zu nehmen. Vorzugsweise werden 1,0 bis 1,3, insbesondere 1,05 bis 1,15 Mol der Säuren je Mol des Alkens angewendet.

Das Mengenverhältnis Alken zu Cyanwasserstoff kann weitgehend beliebig gewählt werden, jedoch ist es in den meisten Fällen vorteilhaft, nicht weniger als etwa 0,8 und nicht mehr als etwa 2,0 Mol Cyanwasserstoff je Mol des Alkens zu nehmen. Vorzugsweise werden 1,0 bis 1,2 Mol Cyanwasserstoff je Mol des Alkens angewendet. Anstelle von Cyanwasserstoff können diesem äquivalente Mengen der Cyanwasserstoff liefernden Substanzen eingesetzt werden. In diesem Fall wird zusätzlich eine entsprechende Menge starker Säuren benötigt.

Zur Durchführung des erfindungsgemässen Verfahrens ist es erforderlich, ein primäres oder sekundäres Alkanol zuzusetzen. Dieses kann als solches oder mit den übrigen Substanzen, gegebenenfalls als Monoester an Schwefelsäure gebunden, eingebracht werden. Wenngleich die Menge dieses Alkanols weitgehend beliebig gewählt werden kann, ist es im allgemeinen zweckmässig, je Mol Alken etwa 1 bis 4 Mol, vorzugsweise 1,1 bis 2,0 Mol des primären oder sekundären Alkanols zu nehmen. Gegebenenfalls wird zu Anfang der Umsetzung nur eine Teilmenge, etwa 0,7 bis 1,2 Mol, und gegen Ende der Umsetzung die Restmenge des primären oder sekundären Alkanols eingebracht.

Zur Durchführung des erfindungsgemässen Verfahrens ist es erforderlich, Wasser zuzusetzen. Dieses kann als solches oder mit den übrigen Substanzen eingebracht werden. Wenngleich die Wassermenge weitgehend beliebig gewählt werden kann, ist es im allgemeinen zweckmässig, nicht mehr als etwa 4 Mol Wasser je Mol Alken zu nehmen. Vorzugsweise werden etwa 0,9 bis 2,0 Mol Wasser je Mol Alken angewendet.

Zweckmässig ist es im allgemeinen, bei Temperaturen etwa zwischen −30 und +100 °C zu arbeiten. Vorteilhaft sind in den meisten Fällen Temperaturen zwischen 20 und 80 °C. Es kann zweckmässig sein, die Umsetzung bei Temperaturen bis zu etwa 50 °C zu beginnen und bei höheren Temperaturen zu beenden, wobei mit Vorteil zunächst Temperaturen gewählt werden, die unterhalb des Siedepunkts des Umsetzungsgemischs liegen, und im weiteren Verlauf das Umsetzungsgemisch zum Sieden gebracht wird.

Wenngleich der Druck weitgehend beliebig gewählt werden kann, ist es vorteilhaft, vom Normaldruck nicht wesentlich abzuweichen. In manchen Fällen kann es wegen des Vorliegens flüchtiger Substanzen zweckmässig sein, bei einem der Temperatur entsprechenden höheren Druck zu arbeiten.

Eine bevorzugte Verfahrensweise ist, die Ausgangssubstanzen, das Alken, das Alkanol, den Cyanwasserstoff und die starke Säure, in wasserfreier oder weitgehend wasserfreier Form einzusetzen, die Umsetzung zunächst bei niederen Temperaturen, die unterhalb des Siedepunktes des Umsetzungsgemischs liegen, durchzuführen, dann das Wasser und gegebenenfalls die restlichen Anteile des Alkanols zuzusetzen und schliesslich das Umsetzungsgemisch zum Sieden zu bringen.

Aus dem Umsetzungsgemisch kann das Amin und der Ester in an sich bekannter Weise abgetrennt und gewonnen werden, wobei sich die Verfahrensweise gegebenenfalls nach der Art der

Substanzen richtet. Vorteilhaft ist es in vielen Fällen, beispielsweise bei der Herstellung des tert.-Butylamins oder tert.-Amylamins einerseits und der Ester der Ameisensäure mit Propanol-(1), Propanol-(2), Äthanol oder Methanol andererseits, zunächst den Ester aus dem Umsetzungsgemisch abzutreiben, dann im restlichen Umsetzungsgemisch mittels alkalisch wirkender Substanzen das Amin freizusetzen und schliesslich dieses abzutreiben.

Beispiel 1

In eine Mischung aus 41,7 g (1,3 Mol) Methanol, 27,0 g (1,0 Mol) Cyanwasserstoff und 56,1 g (1,0 Mol) Isobuten wurden im Verlauf von 30 Minuten 110 g (1,1 Mol) konzentrierte Schwefelsäure eingetragen. Die Mischung wurde währenddessen auf −25 bis −15 °C, danach 60 Minuten lang auf 20 °C und schliesslich 60 Minuten lang auf 50 °C gehalten. Nach Abkühlung auf 20 °C und Zugabe von 12,6 g (0,7 Mol) Wasser wurde der Ameisensäuremethylester (Siedebereich 31 bis 34 °C) abdestilliert. Es wurden 56 g des Esters gewonnen, entsprechend 93% Ausbeute, bezogen auf den eingesetzten Cyanwasserstoff. Aus dem restlichen Umsetzungsgemisch wurde nach Zugabe von 100 g Wasser das Methanol und nach Zugabe von 196 g einer 45%igen wässrigen Natriumhydroxidlösung das tert.-Butylamin (Siedetemperatur 46 °C) abdestilliert. Es wurden 62 g des Amins gewonnen, entsprechend 85% Ausbeute, bezogen auf das eingesetzte Isobuten.

Beispiel 2

Es wurde wie nach Beispiel 1 verfahren, jedoch wurden statt Isobuten 70,1 (1,0 Mol) 2-Methylbuten-(2) eingesetzt. Die Ausbeute an Ameisensäuremethylester betrug 55 g, entsprechend 91%, bezogen auf den eingesetzten Cyanwasserstoff, die Ausbeute an tert.-Amylamin 73 g entsprechend 84%, bezogen auf das eingesetzte 2-Methylbuten-(2). Der Siedepunkt des tert.-Amylamins war 77 °C.

Beispiel 3

Es wurde wie nach Beispiel 1 verfahren, jedoch wurden statt Methanol gleichmolare Mengen Äthanol eingesetzt. Die Ausbeute an Ameisensäureäthylester betrug 63 g, entsprechend 85%, bezogen auf den eingesetzten Cyanwasserstoff, die Ausbeute an tert.-Butylamin 59 g, entsprechend 81%, bezogen auf das eingesetzte Isobuten.

Beispiel 4

Es wurde wie nach Beispiel 1 verfahren, jedoch wurden statt Methanol gleichmolare Mengen Propanol-(2) eingesetzt. Die Ausbeute an Ameisensäureisopropylester betrug 67 g, entsprechend 76%, bezogen auf den eingesetzten Cyanwasserstoff, die Ausbeute an tert.-Butylamin 60 g, entsprechend 82%, bezogen auf das eingesetzte Isobuten.

Beispiel 5

In 40,04 kg 97%ige Schwefelsäure (396 Mol Schwefelsäure) wurde eine Lösung von 9,73 kg (360 Mol) Cyanwasserstoff in 14,88 kg (465 Mol) Methanol und 25,25 kg (360 Mol) 2-Methylbuten-(2) eingespeist. Die Mischung wurde währenddessen auf 20 bis 25 °C und danach 60 Minuten lang auf 50 °C gehalten. Nach Zugabe von 8,00 kg Methanol und 8,52 kg Wasser wurde die Mischung 60 Minuten lang unter Rückfluss auf Siedetemperatur gehalten. Dann wurde der Ameisensäuremethylester abdestilliert. Aus dem restlichen Umsetzungsgemisch wurde nach Zugabe von 70,40 kg einer 45%igen wässrigen Natriumhydroxidlösung das tert.-Amylamin abdestilliert. Die Ausbeute betrug 19,7 kg Ameisensäuremethylester, entsprechend 91%, bezogen auf den eingesetzten Cyanwasserstoff, und 27,5 kg tert.-Amylamin (Reinheit 98%), entsprechend 86%, bezogen auf das eingesetzte 2-Methylbuten-(2).

Beispiel 6

In eine Mischung aus 112,2 g (1,0 Mol) 2,4,4-Trimethylpenten-(1), 41,6 g (1,3 Mol) Methanol und 27,0 g (1,0 Mol) Cyanwasserstoff wurden im Verlauf von 30 Minuten 112,3 g 96%ige Schwefelsäure (1,1 Mol) eingetragen. Die Mischung wurde währenddessen auf 25 °C, danach 60 Minuten lang auf 50 °C und nach Zugabe von 22,4 g Methanol und 22,5 g Wasser 60 Minuten lang unter Rückfluss auf Siedetemperatur gehalten. Dann wurde der Ameisensäuremethylester und nach Zugabe von 100 g Wasser das überschüssige Methanol abgetrieben. Schliesslich wurden 196 g einer 45%igen Natriumhydroxidlösung und 500 g Wasser zugesetzt. Die Mischung wurde dreimal mit Diäthyläther extrahiert, die Ätherextrakte wurden vereinigt, mit Natriumsulfat getrocknet und destilliert. Die Ausbeute betrug 54 g Ameisensäuremethylester entsprechend 90%, bezogen auf den eingesetzten Cyanwasserstoff, und 97 g 1,1,3,3-Tetramethylbutylamin, entsprechend 75%, bezogen auf das eingesetzte 2,4,4-Trimethylpenten-(1). Der Siedepunkt des Amins war 76 °C bei 130 mbar.

Beispiel 7

In eine Mischung aus 96 g (1,0 Mol) 1-Methylcyclohexen, 32 g (1,0 Mol) Methanol und 27 g (1,0 Mol) Cyanwasserstoff wurden 153 g 96%ige Schwefelsäure (1,5 Mol) eingetragen. Die Mischung wurde währenddessen auf 25 °C, danach 60 Minuten lang auf 50 °C und nach Zugabe von 22 g Wasser und 32 g Methanol 60 Minuten lang unter Rückfluss auf Siedetemperatur gehalten. Dann wurde der Ameisensäuremethylester und nach Zugabe von 100 g Wasser das überschüssige Methanol abgetrieben. Dem restlichen Umsetzungsgemisch wurden 267 g einer 45%igen wässrigen Natriumhydroxidlösung zugesetzt. Es bildeten sich 2 Phasen, von denen die obere 1-Methylcyclohexylamin war. Die untere Phase wurde mit Diäthyläther extrahiert, und der Extrakt wurde mit der oberen Phase vereinigt. Diese wurde dann mit Natriumsulfat getrocknet

und zur Gewinnung des Amins destilliert. Der Siedepunkt des Amins war 142 °C. Die Ausbeute betrug 54 g Ameisensäuremethylester, entsprechend 90%, bezogen auf den eingesetzten Cyanwasserstoff, und 86 g 1-Methylcyclohexylamin (Reinheit 93%) entsprechend 71%, bezogen auf das eingesetzte 1-Methylcyclohexen.

## Patentansprüche

1. Verfahren zur Herstellung von N-tert.-Alkylaminen oder Cycloalkylaminen und Estern der Ameisensäure unter Umsetzung von Alkenen mit Cyanwasserstoff und Wasser in Gegenwart starker Säuren, dadurch gekennzeichnet, dass Alkene, die an mindestens einer Seite der Doppelbindung verzweigt sind, oder Cycloalkene in Gegenwart der bis zu etwa zweifachen molaren Menge der starken Säuren mittels primärer oder sekundärer Alkanole zu N-tert.-Alkylaminen oder Cycloalkylaminen und Estern der Ameisensäure mit primären oder sekundären Alkanolen umgesetzt werden.

2. Verfahren nach Anspruch 1, dadurch gekennzeichnet, dass 1,05 bis 1,15 Mol Säure je Mol Alken angewendet wird.

3. Verfahren nach Anspruch 1 oder 2, dadurch gekennzeichnet, dass als Säure Schwefelsäure verwendet wird.

4. Verfahren nach Anspruch 1 bis 3, dadurch gekennzeichnet, dass aus dem Umsetzungsgemisch zuerst der Ameisensäureester und dann das Amin abgetrennt wird.

## Revendications

1. Procédé pour la fabrication de N-tert.-alcoylamines ou de cycloalcoylamines et d'esters de l'acide formique par réaction d'alkènes avec de l'acide cyanhydrique et de l'eau, en présence d'acides forts, caractérisé en ce que l'on fait réagir des alkènes qui sont ramifiés sur un côté au moins de la double liaison, ou des cycloalkènes, en présence d'une quantité qui peut être jusqu'à environ deux fois molaire d'acides forts, au moyen d'alcanols primaires ou secondaires, pour former des N-tert.-alcoylamines ou des cycloalcoylamines et des esters d'acide formique avec des alcanols primaires ou secondaires.

2. Procédé suivant la revendication 1, caractérisé en ce que l'on utilise 1,05 à 1,15 mole d'acide par mole d'alkène.

3. Procédé suivant l'une des revendications 1 et 2, caractérisé en ce que comme acide, on utilise l'acide sulfurique.

4. Procédé suivant l'une quelconque des revendications 1 à 3, caractérisé en ce que l'on sépare d'abord du mélange réactionnel, l'ester d'acide formique et ensuite, l'amine.

## Claims

1. A process for the production of N-tertiary-alkyl amines or cycloalkylamines and esters of formic acid by reacting alkenes with hydrogen cyanide and water in the presence of strong acids, characterised in that alkenes, which are branched on at least one side of the double bond, or cycloalkenes are reacted in the presence of up to about double the molar quantity of the strong acids and in the presence of primary or secondary alkanols to produce N-tertiary-alkylamines or cycloalkylamines and esters of formic acid with primary or secondary alkanols.

2. A process according to claim 1, characterised in that from 1.05 to 1.15 mols of acid are used per mol of alkene.

3. A process according to claim 1 or 2, characterised in that sulphuric acid is used as acid.

4. A process according to claim 1 to 3, characterised in that first of all the formic acid ester is separated, and then the amine is separed from the reaction mixture.